# EUROPEAN PATENT SPECIFICATION

(11) **EP 3 475 860 B1**
(45) Date of publication and mention of the grant of the patent: **07.08.2024**
(21) Application number: 17739321.2
(22) Date of filing: 21.06.2017
(51) Int. Cl.: G16H 30/20, G16H 30/40, G16H 40/63, G16H 50/20

(54) **SYSTEM AND ARCHITECTURE FOR SEAMLESS WORKFLOW INTEGRATION AND ORCHESTRATION OF CLINICAL INTELLIGENCE**
SYSTEM UND ARCHITEKTUR ZUR NAHTLOSEN ARBEITSFLUSSINTEGRATION UND ORCHESTRIERUNG VON KLINISCHER INTELLIGENZ
SYSTÈME ET ARCHITECTURE D'INTÉGRATION HOMOGÈNE DE FLUX DE TRAVAIL ET D'ORCHESTRATION D'INTELLIGENCE CLINIQUE

(30) Priority: 28.06.2016 US 201662355417 P
(43) Date of publication of application: 01.05.2019
(73) Proprietor: Koninklijke Philips N.V., 5656 AG Eindhoven (NL); The University of Chicago, Chicago, IL 60637 (US)
(72) Inventor: CHANG, Paul Joseph, 5656 AE Eindhoven (NL); SEVENSTER, Merlijn, 5656 AE Eindhoven (NL); TAHMASEBI MARAGHOOSH, Amir Mohammad, 5656 AE Eindhoven (NL); VAN OMMERING, Robbert Christiaan, 5656 AE Eindhoven (NL); SHUALI, Aharona, 5656 AE Eindhoven (NL); ZINO, Eliahu, 5656 AE Eindhoven (NL)
(74) Representative: van Oudheusden-Perset, Laure E.
(86) International application number: PCT/IB2017/053690
(87) International publication number: WO 2018/002776

(56) References cited:
- WO-A1-2014/155273
- WO-A1-2015/114485
- US-A1- 2016 124 619
- US-A1- 2016 171 299

## Description

### Background

PACS (Picture Archiving Communications System) has revolutionized the way radiologists work by making workflow more efficient. This has allowed radiologists to read an increasing amount of studies at a faster rate. Further, improved workflow efficiency has led to faster reporting of critical findings, lower rescan rates, more uniformity in imaging scans, clearer actionable reports, enhanced patient experience, and improvements to reimbursements. PACS may offer even more benefits to radiologists.

Despite improvements in radiology workflow, the reading of an imaging exam or study for a patient may still be a difficult and time-consuming process for a radiologist. In some cases, radiologists spend substantial time finding relevant patient exams, selecting appropriate applications in an image processing application, waiting for the applications to finalize computation, recording key outcome findings (e.g., measurements), and inputting relevant information into a radiology report. Thus, there is still room for improvement.

As is well known, the detection, quantification, characterization and diagnosis of individual lesions in radiology exams is subject to substantial intra-rater variability and even more substantial inter-rater variability. These observations negatively affect the perception of Radiology as an exact science, and, more concretely, may degrade the satisfaction of referring physicians with the service of his/her Radiology department of choice.

WO2015114485A1 describes a system for providing actionable annotations includes a clinical database storing one or more clinical documents including clinical data, wherein a context extraction and classification engine generates clinical context information from the clinical data and an annotation recommending engine generates a list of recommended annotations based on the clinical context information.

WO2015114485A1 does not disclose at least the cues of selecting a voxel and/or taking a measurement of a lesion, and hence further improvements are needed.

### Summary

The invention is defined by the independent claims. Dependent claims represent beneficial embodiments.

The invention provides a method, a system, and a computer program product as specified by the independent claims. Preferred embodiments are laid down in the dependent claims.

In one aspect described herein, A method for determining a desired action of a user reading a medical image is described, the comprising: retrieving and displaying an image to be read by a user; receiving, via a processor, a contextual cue of the user in response to the displayed image to be read, wherein the contextual cue comprises selecting, by a user interface, a voxel; monitoring, interpreting and mapping the contextual cue to a user intention via the processor, wherein the user intention comprises acquiring a volumetric quantification of a lesion nearest the voxel selected by the user interface; and generating an action based on the user intention via the processor, wherein the action comprises retrieving a three-dimensional segmentation of the lesion nearest the voxel selected by the user interface, wherein, if a voxel is selected by the user interface, then the user intention is determined to be acquiring volumetric quantification of the lesion nearest the voxel selected by the user interface, and if the user intention is determined to be acquiring volumetric quantification of the lesion nearest the voxel selected by the user interface, then the action is retrieving the three-dimensional segmentation of the lesion nearest the voxel selected by the user interface.

In another aspect, a system for determining a desired action of a user reading a medical image is described. The system comprises: a display configured for displaying an image to be read by a user; a processor configured for receiving a contextual cue of the user in response to the displayed image to be read, wherein the contextual cue comprises selecting, by a user interface, a voxel; a processor configured for monitoring, interpreting and mapping the contextual cue to a user intention via the processor, wherein the user intention includes acquiring a volumetric quantification of a lesion nearest the voxel selected by the user interface, and generating an action based on the user intention via the processor, wherein the action includes retrieving a three-dimensional segmentation of the lesion nearest the voxel selected by the user interface, wherein, if a voxel is selected by the user interface, then the user intention is determined to be acquiring volumetric quantification of the lesion nearest the voxel selected by the user interface, and if the user intention is determined to be acquiring volumetric quantification of the lesion nearest the voxel selected by the user interface, then the action is retrieving the three-dimensional segmentation of the lesion nearest the voxel selected by the user interface.

### Brief Description

Fig. 1 shows a schematic drawing of a system according to an embodiment.
Fig. 2 shows another schematic drawing of the system of Fig. 1.
Fig. 3 shows a schematic drawing of a system according to another embodiment.
Fig. 4 shows a flow diagram of a method according to an embodiment.
Fig. 5 shows a screenshot of a first image including lesion annotations.
Fig. 6 shows a screenshot of the first image including lesion characterizing information.
Fig. 7 shows a screenshot of the first image including a malignancy prediction for a lesion.
Fig. 8 shows a screenshot of the first image including a sentence characterizing and diagnosing the lesion.
Fig. 9 shows a screenshot of a second image including a lesion measurement.
Fig. 10 shows a screenshot of the second image including a 3D overlay on a lesion.
Fig. 11 shows a screenshot of the second image including a cross-sequence of images of the lesion.

### Detailed Description

The embodiments may be further understood with reference to the following description and the appended drawings, wherein like elements are referred to with the same reference numerals.

Advanced image processing (AIP) and automated clinical intelligence (CI) are highly useful tools for radiologists and other medical professionals. However, they suffer from at least two distinct problems. A first problem is a lack of workflow integration. In particular, AIP, because of its computational intensive nature, is typically executed from dedicated workstations that cannot be consulted without the radiologist physically switching desks, logging on, finding the relevant patient exams, selecting the appropriate applications in the AIP environment, waiting for the applications to finalize computation, recording key outcome findings (e.g., measurements), physical moving back to the routine workstations, and, finally, copying relevant information into a radiology report.

The second problem is observer variability. In particular, as is known by those of skill in the art, the detection, quantification, characterization and diagnosis of individual lesions in radiology exams is subject to substantial intra-rater variability and even more substantial inter-rater variability. These observations negatively affect the perception of radiology as an exact science, and more concretely, may downgrade the satisfaction of referring physicians with the service of the radiology department of choice.

The embodiments discussed below provide solutions to these and other problems with AIP. Thus, the embodiments address the issues of the lack of workflow integration and observer variability, which are problems that are rooted in computer technology, and solve them.

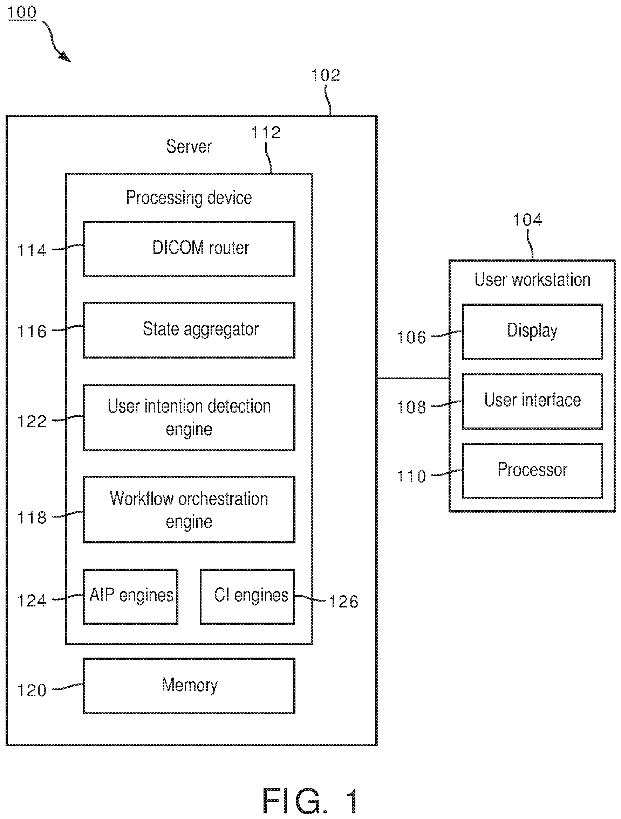

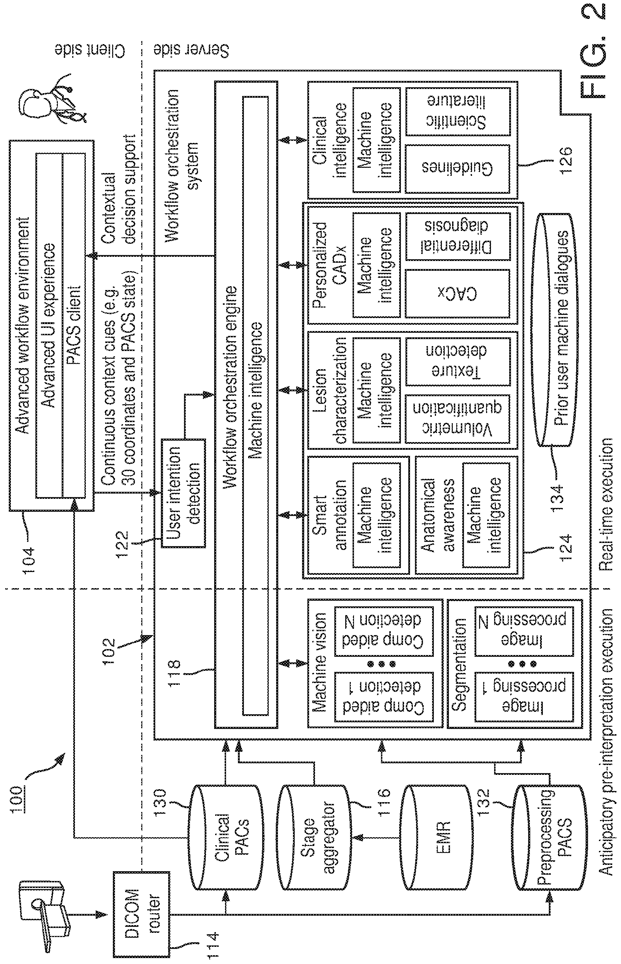

As shown in Figs. 1 and 2, a system 100 according to an embodiment of the present disclosure determines a desired action to be taken by a user reading an image of a patient body segment. The system 100 may comprise a server 102 and a user workstation 104 including, for example, a display 106 and a user interface 108, and a processor 110. The server 102 may further include a processing device 112 including various programs and/or engines such as, for example, a Digital Imaging and Communications in Medicine (DICOM) router 114, a state aggregator 116 and a workflow orchestration engine 118, along with a memory 120. Although the server 102 is shown and described as being connected to a single user workstation 104, it will be understood by those of skill in the art that the server 102 may be connected to any number of workstations 104 on which the images to be read may be reviewed by a user.

The processor 110 may be configured to execute a plurality of applications of the server 102. For example, the applications may include the functionality associated with the workflow orchestration engine 118. The functionality associated with the applications may also be represented as a separate incorporated component of the user workstation 104 or may be a modular component coupled to user workstation 104, e.g., an integrated circuit with or without firmware. For example, the processor 110 may be a hardware component that comprises circuitry necessary to interpret and execute electrical signals fed into the system 100. Examples of processors include central processing units (CPUs), control units, microprocessors, etc. The circuitry may be implemented as an integrated circuit, an application specific integrated circuit (ASIC), etc. The embodiments may be implemented in any of these or other configurations of a user workstation 104.

The display 106 may be a hardware component configured to show data to a user. The display 106 may be, for example, a liquid crystal display (LCD) device, a light emitting diode (LED) display, an organic LED (OLED) display, a plasma display panel (PDP), etc. Those skilled in the art will understand that the functionalities of the user interface 108 and the display 106 may be implemented in a single hardware component. For example, a touchscreen device may be used to implement both the display and the user interface 108.

Disclosure not forming part of the invention may include a DICOM router 114 that receives images to be read, send the images to a Picture Archiving Communications System (PACS), and notify the workflow orchestration engine 118. The PACS may be a workstation that aids the user (e.g. radiologist) in their duties and allows them to keep up with ever increasing workloads. In particular, the PACS provides access to a patient study including, for example, a patient's radiological history, including diagnostic reports, exam notes, clinical history and images. Further, the PACS has features that simplify and speed up workflow to optimize the productivity of a user in reviewing volumes of patient studies in order to maintain a cost-effectiveness and efficiency.

The images received by the DICOM router 114 may, for example, be routed to both a Clinical PACS 130 and a pre-processing PACS 132. The Clinical PACS 130 stores the images to be read until a user retrieves them for reading. The pre-processing PACS 132 may, for example, identify the type of image (e.g., CT, MRI) and the body segment (e.g., lung, brain) that is imaged in an image to be read. This may help streamline the workflow process. The state aggregator 116 extracts patient information that may be pertinent to the image to be read from one or more medical repositories such as, for example, EMR (Electronic Medical Record), Radiology Information Systems, PACS, and/or clinical data feeds, through, for example, Application Programming Interfaces (APIs). The state aggregator 116 may extract information such as, for example, demographic information, active diagnoses, personal and family risk factors, prior surgery events, medications, allergies, etc. The images to be read and any corresponding information such as image type, body segment and patient information may be stored to the memory 120.

Disclosure not forming part of the invention may include a workflow orchestration engine 118 that monitors and interprets contextual cues of a user (e.g., radiologist) reading the image using, for example, a user intention detection engine 122, AIP (Advanced Image Processing) engines 124 and CI (Clinical Intelligence) engines 126. The user intention detection engine 118 receives the contextual cues of the user and maps them to one or more user intentions such as, "acquire volumetric quantification", "acquire anatomical location", acquire view of this region of interest in other sequences of this exam", and "acquire view of this region of interest in relevant prior exam." AIP engines 124 may include, for example, smart annotation, lesion characterization, personalized CADx and anatomical awareness engines, as shown in Fig. 2, which may provide, for example, automated lesion detection, automated lesion characterization, automated lesion segmentation, automated anatomy segmentation, automated abnormality detection and/or automated multi-sequence registration. CI engines 126 may include, for example, automated risk calculation engines, automated guidelines engines, automated diagnoses engines, automated report content generation, and automated retrieval of similar cases. The workflow orchestration engine 118 may receive a user intention object identified by the user intention detection engine 118 and initiate one or more API and/or CI engine 124, 126 via a rule-based logic, which will be described in further detail below. These rules may be stored to the memory 120.

In one aspect, the user workstation 104 may be a personal computing device which, as described above, includes a display 106 for viewing the image to be read and a user interface 108 which may be used for providing the contextual cues to the workflow orchestration engine 116. The user may login at the user workstation, providing user authentication information associated with a professional profile including information such as, for example, a specialty and/or seniority. The user authentication information may be used to store specific user preferences and/or to tailor the predicted desired actions to each user. In some embodiments, a user's specialty or seniority may be used to initiate specific CI engines 126 to provide decision support. For example, where a user is identified as being a junior faculty member, the server 102 may initiate an automated guideline and/or diagnosis engine to aid in providing a proper diagnosis or reading of the image to address the user's lack of experience.

The user may login and provide contextual cues using the user interface 108, which may include input devices such as, for example, a keyboard, a mouse, a touch display on the display 106 and/or an eye movement tracking device. Although the exemplary embodiments describe the contextual cues as being generated via a mouse using, it will be understood by those skilled in the art that the contextual cues may be generated in any of a variety of ways including, for example, eyeball tracking via gestures and/or eye blinks. The processor 110 communicates with the server 102 to retrieve and display images to be read, manipulate the image to be read, provide contextual cues, include findings in a radiology report, etc.

Disclosure not forming part of the invention may include contextual cues generated viathe user interface 108 of the user workstation 104 are provided to the workflow orchestration engine 118, which receives the user intention object and orchestrates one or more AIP and/or CI engines 124, 126. As described above, the dynamic sequencing of engines can be driven by rule-based logic. Examples of rule-based logic may be as follows. If the user intention is determined to be "acquire volumetric quantification", then retrieve the three-dimensional segmentation of the lesion that is nearest the voxel selected by the user interface 108 (e.g., mouse device). If the user intention is determined to be "annotate lesion", then retrieve the anatomical location of the mouse device and funnel the output into the Smart Annotation CI engine. If the output of the Smart Annotation CU equals "nodule" and the anatomical location equals "lung", then run the Lung nodule segmentation AIP engine or retrieve its output if it has already been run in pre-processing mode. If a finding was annotated "nodule" and the anatomical location equals "lung", then retrieve smoking status and age from state aggregator 116 and feed these as input values to the follow-up recommendation engine. If a finding as annotated "nodule", the anatomical location equals "lung" and the user intention is "determine malignancy risk", retrieve age and smoking status from the state aggregator 116, retrieve spiculation characterization and emphysema detection information from the appropriate AIP engines 124 and feed them to the risk model. It will be understood by those of skill in the art that the above outlined rules are exemplary only, and that any number of rules may be instituted to mimic a decision/diagnosis process of a user or to follow accepted guidelines within the industry. In addition, although the exemplary embodiment specifically describes the diagnosis and reading of a lung nodule, the system and method of the present disclosure may be used for the reading/diagnoses of lesions/nodules in any of a variety of body segments and types of images.

Once the user's contextual cues have produced a predicted desired action of the user, the user may modify the results, accept the results or reject the results. Accepting or rejecting the results may indicate to the server 102 whether the workflow orchestration engine 118 properly determined/predicted the desired action of the user. Each user-system dialogue, which may include contextual cues, derived user information, state information retrieved from the state aggregator 116, image information computed by AIP engines 124, decision support computed by CI engines 126 and/or the action of the user in response to the displayed decision support, may be stored to a prior user-machines dialogues database 134 in the memory 120.

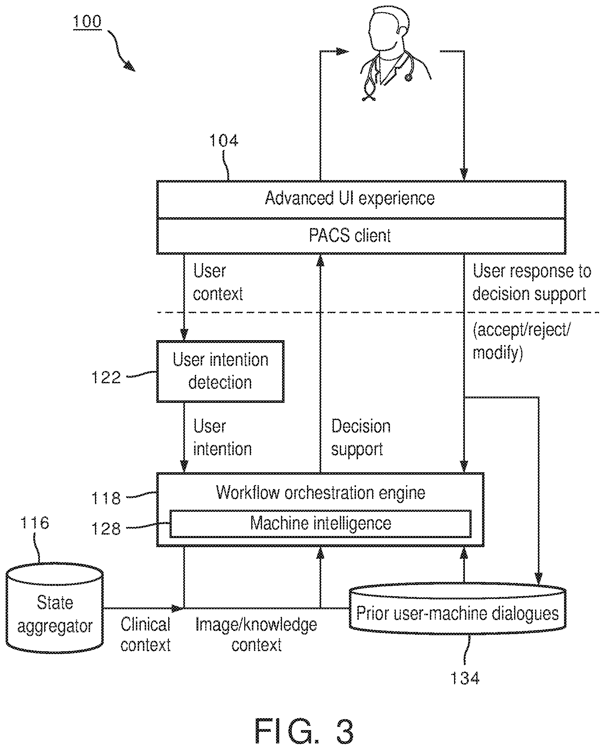

According to a further embodiment, the system 100 may further include a machine intelligence layer 128, also referred to as a machine learning layer, which may access prior user-system dialogues stored in the memory 120 to train the system 100 using machine learning techniques. The machine intelligence layer 128 may be included in the workflow orchestration engine 118, as shown in Fig. 3. The machine intelligence layer 128 may predict the positive user responses to determine whether the system 100 is properly predicting the desired actions of the user and/or providing the proper decision support to the user. For example, where a user accepts an action predicted by the workflow orchestration engine 118, the machine intelligence layer 128 would interpret the acceptance as a positive user response. If the user rejects the predicted action, the machine intelligence layer 128 may interpret the rejection as a negative user response. The workflow orchestration engine 118 may alter the predicted actions and/or provided decision support based on the positive/negative responses of the user.

Disclosure not forming part of the invention may include, each engine (e.g., AIP engines 124, CI engines 126) may include its own machine intelligence layer, as shown in Fig. 2. This machine intelligence layer may be optimized to predict the user's feedback on the output of the individual engines based on the contextual cues and the initial output of the engine itself. When applied on a series of contextual cues and the initial output of the engine, the outcome of the machine intelligence layer may be different from the engine's output. In that case, the machine intelligence layer may overrule the original engine.

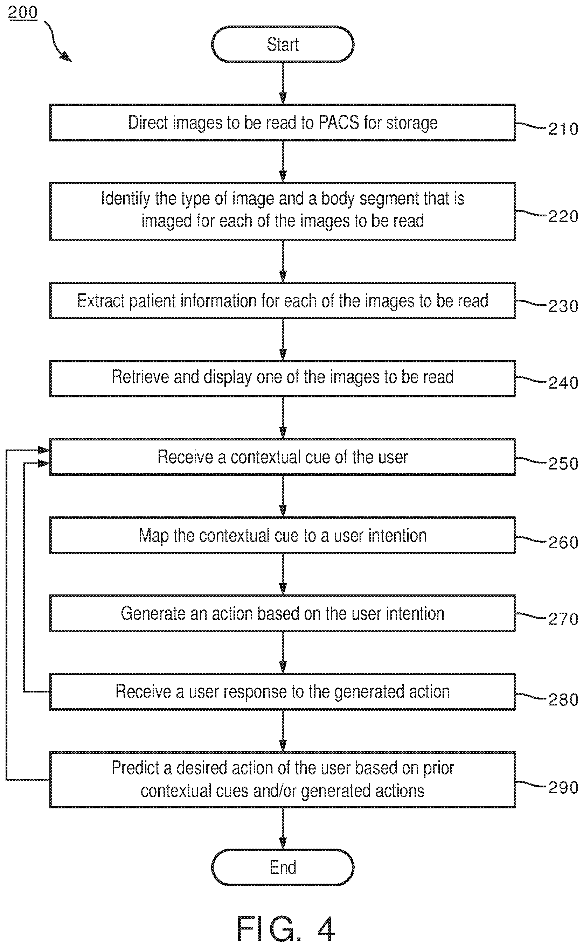

Fig. 4 shows a method 200 according to an embodiment for predicting a desired user action based on contextual cues provided by the user when reading an image. In a step 210, a DICOM router 114 directs images to be read to, for example, the Clinical PACS 130 for storage prior to being retrieved and read by a user and notifies the workflow orchestration engine 118 that the images have been stored and are ready to be read. The DICOM router 114 may also direct the images to be read to the pre-processing PACS so that the type of image and a body segment being imaged may be identified for each of the images to be read, in a step 220. In a step 230, the state aggregator 116 may also extract relevant patient information for each of the images to be read. As described above with respect to the system 100, the patient information may include, for example, demographic information, active diagnoses, personal and family risk factors, prior surgery events, medications, allergies, etc.

Once the images to be read have been processed, as described above, a user may retrieve one of the images to be read, in a step 240, so that the image to be read is displayed on the display 106. As described above, the user may login to the user workstation 104 so that the user profile, which may include authentication information, is also available to the workflow orchestration engine 118. During the reading of the image, the user may provide contextual cues to the server 102 via the user interface 108, in a step 250. The contextual cues may be continuously monitored by the workflow orchestration engine 118. In a step 260, the user intention detection engine 122 maps the detected contextual cue to a user intention. For example, when the user hovers his/her mouse pointer over a lesion in a region of interest on the displayed image, the user intention detection engine 122 may determine that the user's intention is to annotate the lesion. In another example, when the user takes a measurement of the lesion by, for example, drawing a line across a width of the lesion, the user intention detection engine 122 may determine that the user's intention is to acquire a volumetric quantification of the lesion.

Additional examples of contextual cues include zooming in or out of the image, clicking on the image, and creating a rectangular region of interest. In addition, movements of the mouse or other input device can be included as cues, with predetermined semantics, similar to smart phone "swipe" functionality. In an advanced embodiment, if an eye-ball tracker is installed, eye movements can be included in the list of contextual cues. It will be understood by those of skill in the art that any of the contextual cues described above may be preassigned a corresponding user intention.

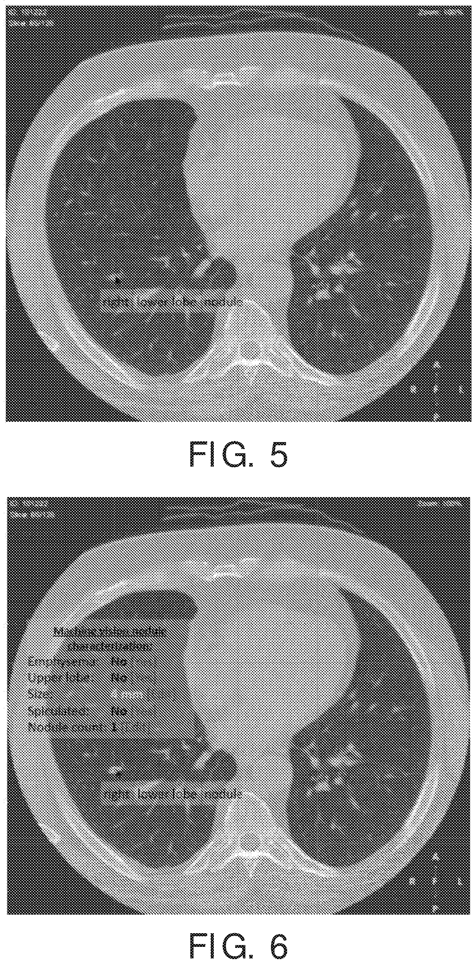

In a step 270, the workflow orchestration engine 118 generates an action based on the user intention. Where the user's intention was determined to be to annotate the lesion, an annotation of the lesion may be displayed over the image. The type of image and body segment identified in the step 220 may be used to annotate the lesion. For example, as shown in Fig. 5, where the image was identified as a CT scan of a lung, the workflow orchestration engine 118 may run, for example, a smart annotation engine of the AIP engines 124, which identifies the lesion as a nodule in the right, lower lobe of the lung and annotates the lesion as such. In another example, as shown in Fig. 6, where the user intention was determined to be to acquire a volumetric quantification of the lesion, the workflow orchestration engine 118 may run a lung nodule segmentation engine of the AIP engines 124, to overlay a 3D volume over the lesion in the displayed image, to determine the three dimensional volume of the lesion. The determined three-dimensional volume, along with any additional available characteristic information (e.g., relevant patient information, spiculation, node count) of the lesion, may be displayed on the display 106.

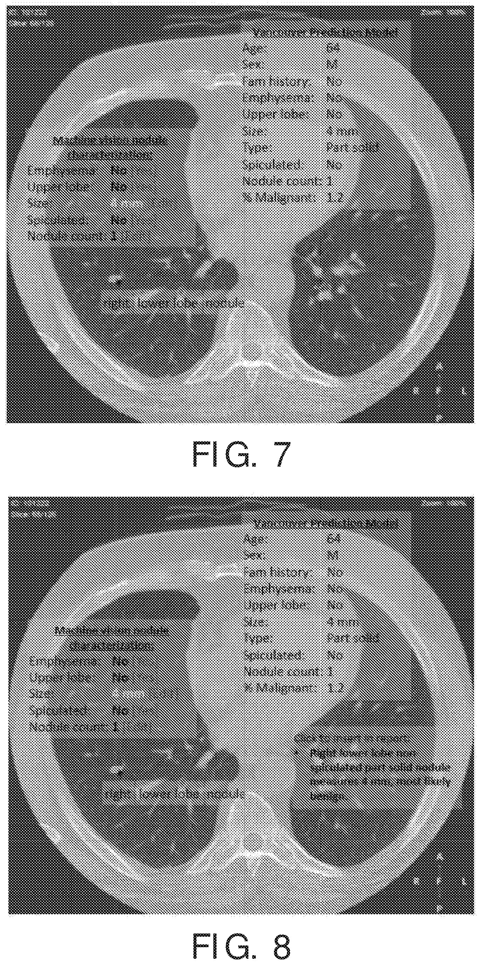

Contextual cues may also include cues that are not directly provided by the user interface 108 (e.g., mouse). For example, a seniority of the user, which may be identified during the user login, may be used to generate decision support actions. In one example, as shown in Fig. 7, where the user is identified as junior faculty, the workflow orchestration engine 118 may run a guideline engine of the CI engines 126 to launch a prediction model such as, for example, the Vancouver prediction model, which estimates malignancy for lung modules. Using previously obtained parameters such as, for example, patient information identified by the state aggregator 116 and a determined size of the lesion, the guideline engine may aid in determining a malignancy of the lesion.

In a step 280, the user may accept, reject or modify the action generated in the step 270. For example, where the annotation was correctly generated, the user may accept the displayed annotation. If the user, however, believes that the annotation was incorrect, the user may reject or modify the annotation. In another example, where the 3D volume overlaid over the lesion is believed to be correct, the user may accept the overlaid volume. If, however, the user believes that the overlaid volume is incorrect, the user may modify contours of the overlaid volume by, for example, clicking and dragging portions thereof, to generate a more accurate three-dimensional quantification of the lesion. In yet another example, where the user believes that the predicted malignancy of the lesion is incorrect, the user may reject or modify the results accordingly. As described above with respect to the system 100, the user's responses to the generated actions may be interpreted via the machine intelligence layer 128 to "train" the system and method regarding the user's desired actions with respect to images being read. Thus, during future readings of images, the system and method may be more accurately able to determine/predict the intentions of the user.

The steps 250 to 280 may be repeated, as necessary, until all of the contextual cues of the user have been interpreted to generate a corresponding predicted action. For example, the workflow orchestration engine 118 may annotate the lesion, determine a three dimensional quantification of the image and provide a predicted malignancy of the lesion during a single reading of the image. In some embodiments, the method 200 may additionally predict further actions desired by the user, in a step 290, based on prior contextual cues and/or generated actions. For example, the workflow orchestration engine 118 may predict that the user may desire to include all of the information derived during the reading of the image to a radiology report for the image. In this example, the workflow orchestration engine 118 may generate and display a sentence characterizing the lesion and providing a diagnosis, as shown in Fig. 8. The user may then copy and paste the sentence into the radiology report or, if an appropriate AIP engine is in place, the sentence may be directly exported to be included in the radiology report.

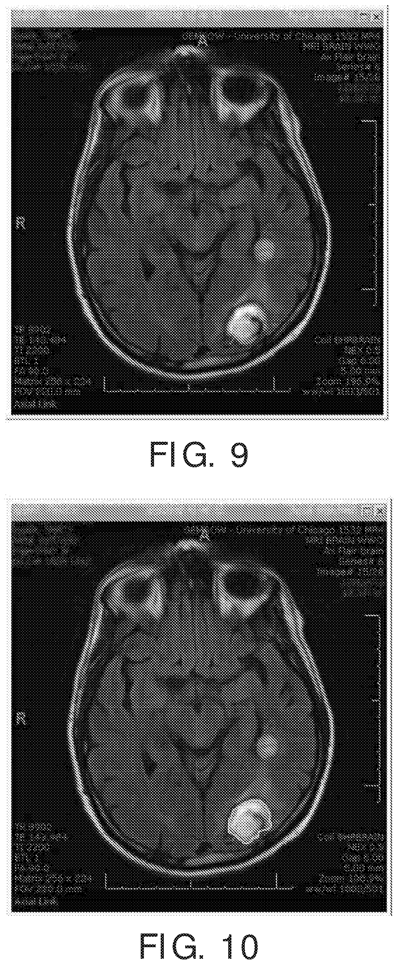

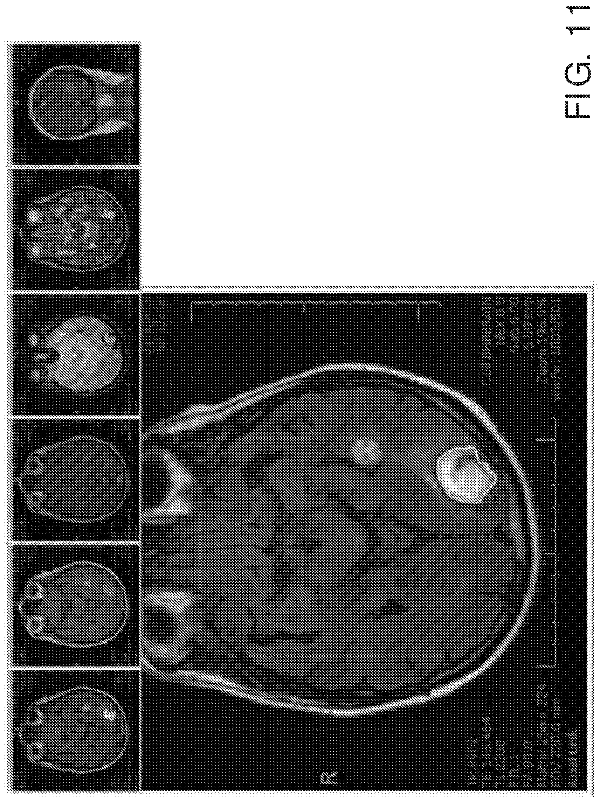

Although the method 200 is described above in regard to a CT scan of a lung nodule, it will be understood by those of skill in the art that the exemplary system and method of the present disclosure may be used during the reading of any of a variety of image types and body segments. In another example, the system 100 and method 200 may be used for the reading of a neurological MRI image. In this example, a user retrieves the image to be read such that it is displayed on the display 106 of the user workstation 104, in the step 240. This image may have been previously stored and processed to identify the type of image and body segment, as well as extract relevant patient information, during the steps 210 - 230. Once the image has been displayed, as shown in Fig. 9, the user may make a linear measurement in a 2D plane of a neurological lesion shown in the displayed image, in the step 250. In the step 260, this contextual cue is mapped to the user intention of acquiring a three dimensional volume of the neurological lesion. In a step 270, a 3D volume overlay is generated over the neurological lesion, as shown in Fig. 10. In the step 280, the user may accept, reject or modify the overlay, substantially as described above in regard to the lung nodule. Based on the prior contextual cues and corresponding generated actions, in the step 290, the workflow orchestration engine 118 may predict that the user is interested in inspecting the lesion cross sequence and generate a ribbon of registered slices across relevant sequences of the image, as shown in Fig. 11, to provide image context to the user.

As described above in regard to the lung nodule example, the steps 250 - 280 and/or 250 - 290 may be repeated as necessary, until all of the desired actions of the user have been generated and applied. Although the exemplary embodiments describe automatically determining/predicting desired actions of the user, it will be understood by those of skill in the art that the user may also manually request that certain actions be taken.

A system 300, according to an exemplary embodiment of the present disclosure, provides contextual interpretation support suggestions to the user. The system 500 comprises the server 102 and the user workstation 104 including, for example, the display 106, the user interface 108, and the processor 110. The exemplary hardware device described above for the server 102 and the user workstation 104 may also be used in the exemplary embodiment. The server 102 may further include the processing device 112 including various programs and/or engines such as, for example, the workflow orchestration engine 118, a sequence normalization engine 130, a sequence registration engine 132, a contextual lesion interpretation support engine 134 and a controller engine 136. Although the server 102 is shown and described as being connected to a single user workstation 104, it will be understood by those of skill in the art that the server 102 may be connected to any number of workstations 104 on which the images to be read may be reviewed by a user. Further, those skilled in the art would understand the system 300 can be completely or partly integrated with the programs and/or engines of system 100.

The workflow orchestration engine 118 may provide contextual cues generated via, for example, the user interface 108 of the user workstation 104. In one example, this may be the radiologist annotating the lesion on the image. The sequence normalization engine 130 may normalize the sequences of the image (or an imaging exam) onto a controlled nomenclature of sequence names. In an exemplary embodiment, the normalization process may take into account information from a sequence header. It should be noted that the account information may be reliable if, for example, modalities at an institution are configured appropriately and/or the user has received proper training. If so, the normalization may be implemented as a table mapping sequence header names onto the controlled nomenclature of sequence names. In another exemplary embodiment, parameter settings from the DICOM router 114 may be leveraged.

The sequence registration engine 132 may register imaging slices across the sequences of at least one of a plurality of imaging exams. For example, the sequence registration engine 132 may register the imaging slices across MRI sequences of one or more distinct MRI exams. In an exemplary embodiment, the registration may be implemented in a PACS viewer, such as, for example, iSite provided by KONINKLIJKE PHILIPS ELECTRONICS N V. In a further exemplary embodiment, for more global matching across the sequences, the registration may be based on taking a whole image volume into account. In another exemplary embodiment, to obtain more localized matching across the sequences, the registration may be based on the lesion in the image.

The contextual lesion interpretation support engine 134 may control a rule base of anatomy-specific questions. The rule base may be managed, for example, by a controller entity that matches the rule base against a structured finding object to determine rules that apply. The controller entity may be, for example, the controller engine 136. When a rule applies, the user may be asked a question(s) and requested to complete the structured finding object. In an exemplary embodiment, each of the questions may be labeled as "Suggestion" or "Mandatory." As such, the user may either be allowed to skip the question or may not have the option to skip the question. Those of skill in the art would understand that the questions may be marked in any manner desired.

In another exemplary embodiment, the rule base may be organized as a decision tree. This may be advantageous since, at any given moment, only one rule applies. In the event the rule is organized as a collection of rules, the controller engine 136 may prioritize which rule to instantiate. For example, the controller engine 136 may prioritize which of the collection of rules to instantiate by using a pre-defined rank.

Each of the questions may be determined based on answers given to previous questions. Additionally, each of the questions may be further determined by content of a structured annotation of the lesion. If, for example, information was already provided through the annotation available in the structured finding object, any questions that can be answered by the information already provided through the annotation are skipped. The instantiated rule may reflect the information desired to be present in any structured finding object, based on previously entered values. In an exemplary embodiment, the decision tree may have decision nodes. The decision nodes may be determined by, for example, contextual information, such as exam modality and anatomy.

In a further exemplary embodiment, each of the questions shown to the user may be accompanied by at least one cross-sequence visualizations of a finding and at least one predetermined answer. Here, for example, the annotated finding may be registered using the sequence registration engine 132 to generate an optimal view on an annotated area of interest.

In another exemplary embodiment, an information button may be utilized to generate background material. The background material may be indexed with, for example, the structured finding objects. Additionally, the background material may provide a presentation of relevant information. For example, the background information may display a medium sized brain tumor that is enhancing and another medium sized brain tumor that is not enhancing.

A method 400 according to an exemplary embodiment provides contextual interpretation support suggestions to the user. In step 410, user interface 108 receives a contextual cue to the image from the user. As discussed above, the contextual cue may be, for example, the radiologist annotating the lesion on the image. In step 420, the sequence normalization engine 130 may normalize the sequences of the image. In step 430, the sequence registration engine 132 may register the imaging slices across the sequences of at least one of the plurality of imaging exams. In step 440, the contextual lesion interpretation support engine 134 may provide contextual lesion interpretation support suggestions to the user. As discussed above, the support suggestions may be the rule base of anatomy specific questions.

It is noted that the claims may include reference signs/numerals. However, the present claims should not be considered to be limited to the exemplary embodiments corresponding to the reference signs/numerals.

Those skilled in the art will understand that the above-described exemplary embodiments may be implements in any number of manners, including, as a separate software module, as a combination of hardware and software, etc. For example, the state aggregator 116, the workflow orchestration engine 118, the user intention detection engine 122, the AIP engines 124, and the CI engines 126 may be programs containing lines of code that, when compiled, may be executed on a processor.

## Claims

1. A method (100) for determining a desired action of a user reading a medical image, comprising:
retrieving and displaying an image to be read by a user;
receiving, via a processor (110), a contextual cue of the user in response to the displayed image to be read, wherein the contextual cue comprises selecting, by a user interface (108), a voxel;
monitoring, interpreting and mapping the contextual cue to a user intention via the processor, wherein the user intention comprises acquiring a volumetric quantification of a lesion nearest the voxel selected by the user interface; and
generating an action based on the user intention via the processor, wherein the action comprises retrieving a three-dimensional segmentation of the lesion nearest the voxel selected by the user interface,
wherein, if a voxel is selected by the user interface, then the user intention is determined to be acquiring volumetric quantification of the lesion nearest the voxel selected by the user interface, and if the user intention is determined to be acquiring volumetric quantification of the lesion nearest the voxel selected by the user interface, then the action is retrieving the three-dimensional segmentation of the lesion nearest the voxel selected by the user interface.

2. The method of claim 1, further comprising:
identifying one of a type of image and a body segment of the image to be read and extracting (230) patient information for the image to be read.

3. The method of claim 2, wherein the patient information includes one of demographic information, active diagnoses, personal and family risk factors, prior surgery events, medications and allergies.

4. The method of any of the preceding claims, further comprising:
receiving a user response to the generated action.

5. The method of claim 4, wherein the user response is one of accepting, rejecting and modifying the generated action.

6. The method of claim 4, further comprising training a machine learning layer (128) of the processor based on the user response, the machine learning layer altering a further generated action based on the user response, wherein accepting the generated action indicates a positive user response, and one of rejecting and modifying the generated action indicates a negative user response.

7. The method of any of the preceding claims, further comprising:
predicting a further desired action of the user based on one of the contextual cue and the generated action.

8. The method of any of the preceding claims, wherein the contextual cue is provided by a user interface.

9. The method of any of the preceding claims, wherein the contextual cue is determined via a user profile.

10. A system (100) for determining a desired action of a user reading a medical image, comprising:
a display (106) configured for displaying an image to be read by a user;
a processor (110) configured for receiving a contextual cue of the user in response to the displayed image to be read, wherein the contextual cue comprises selecting, by a user interface, a voxel;
the processor (110) further configured for
monitoring, interpreting and mapping the contextual cue to a user intention via the processor, wherein the user intention includes acquiring a volumetric quantification of a lesion nearest the voxel selected by the user interface, and
generating an action based on the user intention via the processor, wherein the action includes retrieving a three-dimensional segmentation of the lesion nearest the voxel selected by the user interface,
wherein, if a voxel is selected by the user interface, then the user intention is determined to be acquiring volumetric quantification of the lesion nearest the voxel selected by the user interface, and if the user intention is determined to be acquiring volumetric quantification of the lesion nearest the voxel selected by the user interface, then the action is retrieving the three-dimensional segmentation of the lesion nearest the voxel selected by the user interface.

11. The system of claim 10,
the processor configured for identifying (220) one of a type
of image and a body segment of the image to be read and extracting patient information for the image to be read, and/or
wherein the patient information includes one of demographic information, active diagnoses, personal and family risk factors, prior surgery events, medications and allergies.

12. The system of any of claims 10 or 11, the processor configured for receiving a user response to the generated action.

13. The system of claim 12,
wherein the user response is one of accepting, rejecting and modifying the generated action, and/or
the processor configured for training a machine learning layer of the processor based on the user response, the machine learning layer altering a further generated action based on the user response, wherein accepting the generated action indicates a positive user response, and one of rejecting and modifying the generated action indicates a negative user response.

14. The system of any of claims 10 to 13,
the processor configured for predicting a further desired action of the user based on one of the contextual cue and the generated action, and/or
wherein the contextual cue is provided by a user interface, and/or
wherein the contextual cue is determined via a user profile.

15. A computer program product comprising instructions which, when the program is executed by a computer, cause the computer to carry out the method of claims 1 to 9.

## Patentansprüche

1. Verfahren (100) zum Bestimmen einer gewünschten Aktion eines Benutzers beim Lesen eines medizinischen Bildes, umfassend:
Abrufen und Anzeigen eines Bildes zum Lesen durch einen Benutzer;
Empfangen eines Kontexthinweises des Benutzers als Reaktion auf das angezeigte, zu lesende Bild über einen Prozessor (110), wobei der Kontexthinweis Auswählen eines Voxels durch eine Benutzerschnittstelle (108) umfasst;
Überwachen, Interpretieren und Zuordnen des Kontexthinweises zu einer Benutzerabsicht über den Prozessor, wobei die Benutzerabsicht Erfassen einer volumetrischen Quantifizierung einer Läsion umfasst, welche dem von der Benutzerschnittstelle ausgewählten Voxel am nächsten liegt; und
Erzeugen einer Aktion basierend auf der Benutzerabsicht über den Prozessor, wobei die Aktion Abrufen einer dreidimensionalen Segmentierung der Läsion umfasst, welche dem von der Benutzerschnittstelle ausgewählten Voxel am nächsten liegt,
wobei, wenn ein Voxel durch die Benutzerschnittstelle ausgewählt wird, die Benutzerabsicht dann dazu bestimmt wird, eine volumetrische Quantifizierung der Läsion zu erfassen, welche dem durch die Benutzerschnittstelle ausgewählten Voxel am nächsten ist, und wenn die Benutzerabsicht dazu bestimmt wird, eine volumetrische Quantifizierung der Läsion zu erfassen, welche dem durch die Benutzerschnittstelle ausgewählten Voxel am nächsten ist, die Aktion dann darin besteht, die dreidimensionale Segmentierung der Läsion abzurufen, welche dem durch die Benutzerschnittstelle ausgewählten Voxel am nächsten ist.

2. Verfahren nach Anspruch 1, weiter umfassend:
Identifizieren eines von einem Bildtyp und einem Körpersegment des zu lesenden Bildes und Extrahieren (230) von Patienteninformationen für das zu lesende Bild.

3. Verfahren nach Anspruch 2, wobei die Patienteninformationen eines von demografischen Informationen, aktuellen Diagnosen, persönlichen und familiären Risikofaktoren, früheren chirurgischen Eingriffen, Medikamenten und Allergien beinhalten.

4. Verfahren nach einem der vorstehenden Ansprüche, weiter umfassend:
Empfangen einer Benutzerreaktion auf die erzeugte Aktion.

5. Verfahren nach Anspruch 4, wobei die Benutzerreaktion eines von Akzeptieren, Ablehnen oder Ändern der erzeugten Aktion ist.

6. Verfahren nach Anspruch 4, weiter Trainieren einer Maschinenlernenebene (128) des Prozessors basierend auf der Benutzerreaktion umfassend, wobei die Maschinenlernenebene basierend auf der Benutzerreaktion eine weitere erzeugte Aktion ändert, wobei Akzeptieren der erzeugten Aktion eine positive Benutzerreaktion anzeigt und eines von Ablehnen und Ändern der erzeugten Aktion eine negative Benutzerreaktion anzeigt.

7. Verfahren nach einem der vorstehenden Ansprüche, weiter umfassend:
Vorhersagen einer weiteren gewünschten Aktion des Benutzers basierend auf einem von dem Kontexthinweis und der erzeugten Aktion.

8. Verfahren nach einem der vorstehenden Ansprüche, wobei der Kontexthinweis durch eine Benutzerschnittstelle bereitgestellt wird.

9. Verfahren nach einem der vorstehenden Ansprüche, wobei der Kontexthinweis über ein Benutzerprofil bestimmt wird.

10. System (100) zum Bestimmen einer gewünschten Aktion eines Benutzers beim Lesen eines medizinischen Bildes, umfassend:
ein Display (106), welches zum Anzeigen eines von einem Benutzer zu lesenden Bildes konfiguriert ist;
einen Prozessor (110), welcher zum Empfangen eines Kontexthinweises des Benutzers als Reaktion auf das angezeigte, zu lesende Bild konfiguriert ist, wobei der Kontexthinweis Auswählen eines Voxels durch eine Benutzerschnittstelle umfasst;
wobei der Prozessor (110) weiter dazu konfiguriert ist,
den Kontexthinweis über den Prozessor zu überwachen, zu interpretieren und einer Benutzerabsicht zuzuordnen, wobei die Benutzerabsicht Erfassen einer volumetrischen Quantifizierung einer Läsion beinhaltet, welche dem von der Benutzerschnittstelle ausgewählten Voxel am nächsten liegt; und
eine Aktion basierend auf der Benutzerabsicht über den Prozessor zu erzeugen, wobei die Aktion Abrufen einer dreidimensionalen Segmentierung der Läsion beinhaltet, welche dem von der Benutzerschnittstelle ausgewählten Voxel am nächsten liegt,
wobei, wenn ein Voxel durch die Benutzerschnittstelle ausgewählt wird, die Benutzerabsicht dann dazu bestimmt wird, eine volumetrische Quantifizierung der Läsion zu erfassen, welche dem durch die Benutzerschnittstelle ausgewählten Voxel am nächsten ist, und wenn die Benutzerabsicht dazu bestimmt wird, eine volumetrische Quantifizierung der Läsion zu erfassen, welche dem durch die Benutzerschnittstelle ausgewählten Voxel am nächsten ist, die Aktion dann darin besteht, die dreidimensionale Segmentierung der Läsion abzurufen, welche dem durch die Benutzerschnittstelle ausgewählten Voxel am nächsten ist.

11. System nach Anspruch 10,
wobei der Prozessor zum Identifizieren (220) eines von einem Bildtyp und einem Körpersegment des zu lesenden Bildes und Extrahieren von Patienteninformationen für das zu lesende Bild konfiguriert ist, und/oder
wobei die Patienteninformationen eines von demografischen Informationen, aktuellen Diagnosen, persönlichen und familiären Risikofaktoren, früheren chirurgischen Eingriffen, Medikamenten und Allergien beinhalten.

12. System nach einem der Ansprüche 10 oder 11, wobei der Prozessor zum Empfangen einer Benutzerreaktion auf die erzeugte Aktion konfiguriert ist.

13. System nach Anspruch 12,
wobei die Benutzerreaktion eines von Akzeptieren, Ablehnen oder Ändern der erzeugten Aktion ist, und/oder
der Prozessor zum Trainieren einer Maschinenlernenebene des Prozessors basierend auf der Benutzerreaktion konfiguriert ist, wobei die Maschinenlernenebene basierend auf der Benutzerreaktion eine weitere erzeugte Aktion ändert, wobei Akzeptieren der erzeugten Aktion eine positive Benutzerreaktion anzeigt und eines von Ablehnen und Ändern der erzeugten Aktion eine negative Benutzerreaktion anzeigt.

14. System nach einem der Ansprüche 10 bis 13,
wobei der Prozessor zum Vorhersagen einer weiteren gewünschten Aktion des Benutzers basierend auf einem von dem Kontexthinweis und der erzeugten Aktion konfiguriert ist, und/oder
wobei der Kontexthinweis durch eine Benutzerschnittstelle bereitgestellt wird, und/oder
wobei der Kontexthinweis über ein Benutzerprofil bestimmt wird.

15. Computerprogrammprodukt, umfassend Anweisungen, die, wenn das Programm von einem Computer ausgeführt wird, den Computer veranlassen, das Verfahren nach den Ansprüchen 1 bis 9 auszuführen.

## Revendications

1. Procédé (100) pour déterminer une action souhaitée d'un utilisateur lisant une image médicale, comprenant :
la récupération et l'affichage d'une image qui doit être lue par un utilisateur ;
la réception, par le biais d'un processeur (110), d'un indice contextuel de l'utilisateur en réponse à l'image affichée à lire, dans lequel l'indice contextuel comprend la sélection, par une interface utilisateur (108), d'un voxel ;
la surveillance, l'interprétation et le mappage de l'indice contextuel à une intention de l'utilisateur par le biais du processeur, dans lequel l'intention de l'utilisateur comprend l'acquisition d'une quantification volumétrique d'une lésion la plus proche du voxel sélectionné par l'interface utilisateur ; et
la génération d'une action basée sur l'intention de l'utilisateur par le biais du processeur, dans lequel l'action comprend la récupération d'une segmentation tridimensionnelle de la lésion la plus proche du voxel sélectionné par l'interface utilisateur,
dans lequel, si un voxel est sélectionné par l'interface utilisateur, alors l'intention de l'utilisateur est déterminée comme étant l'acquisition d'une quantification volumétrique de la lésion la plus proche du voxel sélectionné par l'interface utilisateur, et, si l'intention de l'utilisateur est déterminée comme étant l'acquisition d'une quantification volumétrique de la lésion la plus proche du voxel sélectionné par l'interface utilisateur, alors l'action est la récupération de la segmentation tridimensionnelle de la lésion la plus proche du voxel sélectionné par l'interface utilisateur.

2. Procédé selon la revendication 1, comprenant en outre :
l'identification de l'un d'un type d'image et d'un segment corporel de l'image à lire et l'extraction (230) d'informations de patient pour l'image à lire.

3. Procédé selon la revendication 2, dans lequel les informations de patient incluent des informations démographiques, des diagnostics actifs, des facteurs de risque personnels et familiaux, des événements chirurgicaux antérieurs, des médicaments et des allergies.

4. Procédé selon l'une quelconque des revendications précédentes, comprenant en outre :
la réception d'une réponse d'utilisateur à l'action générée.

5. Procédé selon la revendication 4, dans lequel la réponse d'utilisateur est l'un de l'acceptation, du rejet et de la modification de l'action générée.

6. Procédé selon la revendication 4, comprenant en outre l'entraînement d'une couche d'apprentissage automatique (128) du processeur sur la base de la réponse d'utilisateur, la couche d'apprentissage automatique altérant une autre action générée sur la base de la réponse d'utilisateur, dans lequel l'acceptation de l'action générée indique une réponse d'utilisateur positive, et l'un du rejet et de la modification de l'action générée indique une réponse d'utilisateur négative.

7. Procédé selon l'une quelconque des revendications précédentes, comprenant en outre :
la prédiction d'une autre action souhaitée de l'utilisateur sur la base de l'un de l'indice contextuel et de l'action générée.

8. Procédé selon l'une quelconque des revendications précédentes, dans lequel l'indice contextuel est fourni par une interface utilisateur.

9. Procédé selon l'une quelconque des revendications précédentes, dans lequel l'indice contextuel est déterminé par le biais d'un profil d'utilisateur.

10. Système (100) pour déterminer une action souhaitée d'un utilisateur lisant une image médicale, comprenant :
un dispositif d'affichage (106) configuré pour afficher une image qui doit être lue par un utilisateur ;
un processeur (110) configuré pour recevoir un indice contextuel de l'utilisateur en réponse à l'image affichée à lire, dans lequel l'indice contextuel comprenant la sélection, par une interface utilisateur, d'un voxel ;
le microprocesseur (110) étant en outre configuré pour :
surveiller, interpréter et mapper l'indice contextuel à une intention de l'utilisateur par le biais du processeur, dans lequel l'intention de l'utilisateur inclut l'acquisition d'une quantification volumétrique d'une lésion la plus proche du voxel sélectionné par l'interface utilisateur, et
générer une action basée sur l'intention de l'utilisateur par le biais du processeur, dans lequel l'action inclut la récupération d'une segmentation tridimensionnelle de la lésion la plus proche du voxel sélectionné par l'interface utilisateur,
dans lequel, si un voxel est sélectionné par l'interface utilisateur, alors l'intention de l'utilisateur est déterminée comme étant l'acquisition d'une quantification volumétrique de la lésion la plus proche du voxel sélectionné par l'interface utilisateur, et, si l'intention de l'utilisateur est déterminée comme étant l'acquisition d'une quantification volumétrique de la lésion la plus proche du voxel sélectionné par l'interface utilisateur, alors l'action est la récupération de la segmentation tridimensionnelle de la lésion la plus proche du voxel sélectionné par l'interface utilisateur.

11. Système selon la revendication 10,
le processeur étant configuré pour identifier (220) l'un d'un type d'image et d'un segment corporel de l'image à lire et extraire des informations de patient pour l'image à lire, et/ou
dans lequel les informations de patient incluent l'un d'informations démographiques, de diagnostics actifs, de facteurs de risque personnels et familiaux, d'événements chirurgicaux antérieurs, de médicaments et d'allergies.

12. Système selon l'une quelconque des revendications 10 et 11, le processeur étant configuré pour recevoir une réponse d'utilisateur à l'action générée.

13. Système selon la revendication 12,
dans lequel la réponse d'utilisateur est l'un de l'acceptation, du rejet et de la modification de l'action générée, et/ou
le processeur est configuré pour entraîner une couche d'apprentissage automatique du processeur sur la base de la réponse d'utilisateur, la couche d'apprentissage automatique altérant une autre action générée sur la base de la réponse d'utilisateur, dans lequel l'acceptation de l'action générée indique une réponse d'utilisateur positive et l'un du rejet et de la modification de l'action générée indique une réponse d'utilisateur négative.

14. Système selon l'une quelconque des revendications 10 à 13,
le processeur étant configuré pour prédire une autre action souhaitée de l'utilisateur sur la base de l'un de l'indice contextuel et de l'action générée, et/ou
dans lequel l'indice contextuel est fourni par une interface utilisateur, et/ou
dans lequel l'indice contextuel est déterminé par le biais d'un profil d'utilisateur.

15. Produit programme d'ordinateur comprenant des instructions qui, lorsque le programme est exécuté par un ordinateur, amènent l'ordinateur à réaliser le procédé selon les revendications 1 à 9.
